# EUROPEAN PATENT SPECIFICATION

(11) **EP 1 746 981 B1**
(45) Date of publication and mention of the grant of the patent: **13.08.2008**
(21) Application number: 05715569.9
(22) Date of filing: 25.02.2005
(51) Int. Cl.: A61K 9/72, A61K 31/4704, B65D 83/14

(54) **MEDICINAL AEROSOL FORMULATION PRODUCTS WITH IMPROVED CHEMICAL STABILITY**
MEDIZINISCHE AEROSOLFORMULIERUNGEN MIT VERBESSERTER CHEMISCHER STABILITÄT
PRODUITS DE FORMULATION D'AEROSOL MEDICINALE A STABILITE CHIMIQUE AMELIOREE

(30) Priority: 13.05.2004 EP 04011425
(43) Date of publication of application: 31.01.2007
(73) Proprietor: CHIESI FARMACEUTICI S.p.A., I-43100 Parma (IT)
(72) Inventor: MEAKIN, Brian, I-43100 Parma (IT); LEWIS, David, I-43100 Parma (IT); JOHNSON, Robert, I-43100 Parma (IT); CHURCH, Tanya, I-43100 Parma (IT)
(74) Representative: Adam, Holger
(86) International application number: PCT/EP2005/002041
(87) International publication number: WO 2005/112902

(56) References cited:
- EP-A- 1 157 689
- WO-A-03/074025
- WO-A-03/078538
- WO-A-20/05084640

## Description

The present invention relates to medicinal aerosol formulation products and, in particular, to aerosol products such as metered dose inhalers (MDls) for delivery of aerosol formulations containing an active ingredient subject to degradation over time when stored in the metered dose inhaler. Metered dose inhalers are, at present, the most efficient and best accepted means for accurately delivering drugs in small doses to the human respiratory tract. Therapeutic agents commonly delivered by the inhalation root include β₂ adrenergic agonist bronchodilators, in particular long acting β₂ agonists.

MDls comprise a pressure resistant aerosol canister typically filled with a product such as a drug dissolved in a liquefied propellant or micronized particles suspended in a liquefied propellant where the container is fitted with a metering valve. Actuation of the metering valve allows a small portion of the spray product to be released whereby the pressure of the liquefied propellant carries the dissolved or micronized drug particles out of the container to the patient. The valve actuator is used to direct the aerosol spray into the patient's oropharynx.

Generally, the valve includes a rubber valve seal (a diaphragm or gasket) intended to allow reciprocal movement of the valve stem while preventing leakage of propellant from the container.

Said rubber valve seals are commonly made of elastomeric material based on the traditional technology of vulcanising a synthetic or natural rubber polymer.

In some documents of the prior art and for example in WO 93/11743 page 8, lines 4-9, WO 02/02167 from page 13, line 16 to page 14, line 23, halobutyl or butyl rubbers are indifferently described together with other elastomeric material such as low density polyethylene, black and white butadiene-acrylonitrile rubbers, neoprene and many others as materials for gaskets to be used in the valves for metered dose inhalers pressurised by hydrofluorocarbon (HFA or HFC) propellants.

On the contrary, in EP 708805 it was stated that conventional devices involving diaphragms [i.e. gaskets or sealing rings] of neoprene (polychloroprene), butyl rubber or butadiene-acrylonitrile "buna" rubbers allow substantial leakage of HFC-134a or HFC-227 from some formulations over time. This leakage can cause a substantial increase in concentration of the active ingredient in the formulation, resulting in delivery of improper dose. Furthermore in some formulations the valve stem tends to stick, pause, or drag during the actuation cycle. To solve these problems in EP 708 805 a diaphragm material stable to dimensional changes when exposed to HFC-134 comprising an ethylene-propylene-diene (EPDM) rubber has been provided.

Recently in WO 03/078538 in the name of Bespak a seal for a valve for use in a pharmaceutical dispensing device formed by a particular elastomeric composition comprising one or more of polyisobutylene, polybutene, butyl rubber, halogenated butyl rubber and derivatives has been claimed. The particular elastomeric composition in fact comprises an isobutylene polymer or co-polymer, a cross linking agent, and an accelerator for the crosslinking agent wherein the accelerator includes a polysulphide compound derived from a dithiocarbonic acid or derivative thereof.

The technical problem underlying the present invention is to provide a medicinal aerosol formulation product, in particular a metered dose inhaler (MDI) for delivery of aerosol formulations, wherein the chemical stability of a preferred class of long acting β₂ agonists as therapeutic agents contained in the aerosol formulation to be delivered by a metered dose inhaler is improved, i.e. the life time of the medicinal aerosol formulation product for delivery of aerosol formulations containing such kind of active ingredients is prolonged.

This technical problem is solved according to the present invention by a medicinal aerosol formulation product with improved chemical stability, comprising a pressurized metered dose inhaler, comprising an aerosol canister equipped with a metering valve provided with sealing rings and/or gaskets made of a vulcanisate of an elastomeric composition of a butyl rubber, a cross-linking agent for the butyl rubber, and an accelerator for the cross-linking agent, wherein the accelerator includes a polysulphide compound derived from a substituted dithiocarbonic acid or derivative thereof,
wherein the pressurized metered dose inhaler contains in the aerosol canister a medicinal aerosol formulation containing
a long acting β₂ agonist,
a hydrofluorocarbon propellant,
a co-solvent, and
a mineral acid as a stabilizer for the active ingredient.
Examples of suitable mineral acids are hydrochloric, phosphoric, nitric and sulfuric acid.

Figure 1 shows a typical metered dose inhaler of the prior art.

Figure 2a shows a metering valve for inverted use (Valois, France).

Figure 2b shows a metering valve for upright use (Bespak, UK).

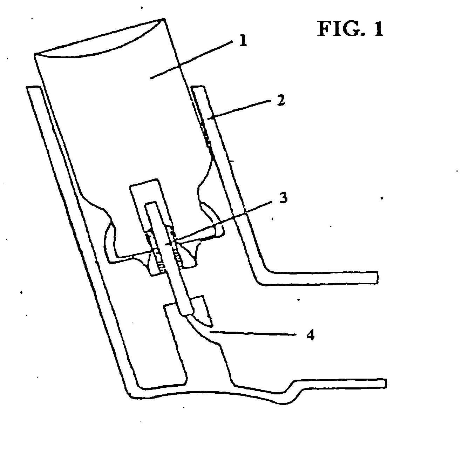

With reference to Figure 1, a typical metered dose inhaler comprises a canister (1), an actuator (2), a metering valve (3) and an actuator orifice (4).

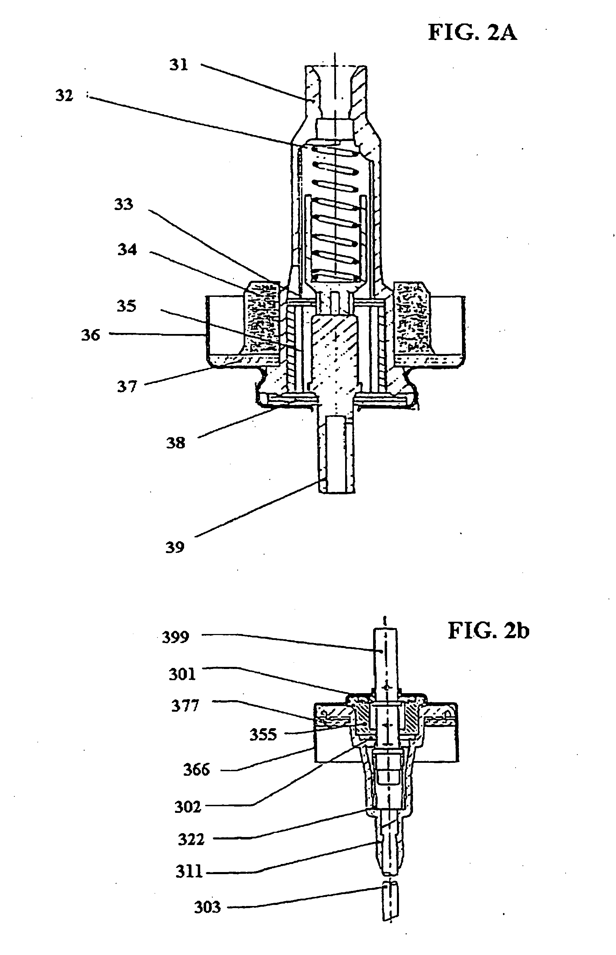

The metering valve should deliver accurately a measured amount of product that should be reproducible not only for each dose delivered from the same package, but from package to package. Two basic types of metering valves are available, one for inverted use (see Figure 2a) and the other for upright use (see Figure 2b). Generally, valves for upright use contain a thin capillary dip tube (303) and are used with solution type aerosols. On the other hand, suspension or dispersion aerosols use a valve for inverted use, which does not contain a dip tube. Figures 2a and 2b illustrate both types of valves and are typical for those commercially available.

In Figures 2a and 2b, the reference signs have the following meanings:
Figure 2a:

| | | |
|---|---|---|
| 31 | = | housing/body |
| 32 | = | spring |
| 33 | = | metering gasket |
| 34 | = | inside gathering ring |
| 35 | = | metering chamber |
| 36 | = | ferrule |
| 37 | = | sealing gasket |
| 38 | = | diaphragm/stem gasket |
| 39 | = | stem |

Figure 2b:

| | | |
|---|---|---|
| 301 | = | seat |
| 302 | = | seat |
| 303 | = | dip tube |
| 311 | = | body |
| 322 | = | spring |
| 355 | = | metering chamber |
| 366 | = | ferrule |
| 377 | = | gasket |
| 399 | = | stem |

The problem with respect to β₂ adrenergic agonist bronchodilators commonly delivered by metered dose inhalers is the instability of these therapeutically active agents in the formulations contained in the metered dose inhalers.

Thus, a problem underlying the present invention is to provide a medicinal aerosol formulation product with improved chemical stability of the long acting β₂ agonist contained in the aerosol formulation as the active ingredient.

The preferred class of long acting β₂ agonists is represented by the formula (I) 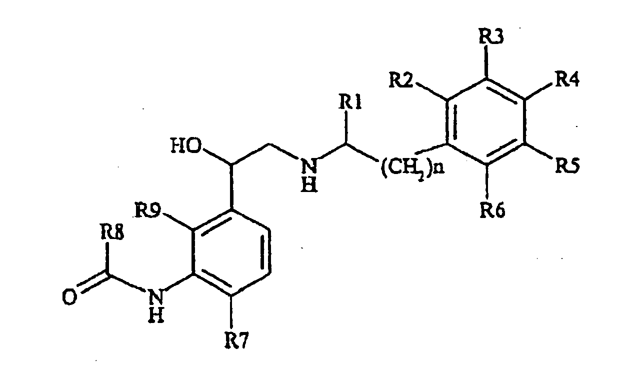 wherein R₁ is methyl and R₂ is hydrogen or R₁ and R₂ form a methylenic bridge -(CH₂)ₙ- with n is 1 or 2,
R₃, R₄, R₅ and R₆ are each independently hydrogen, hydroxy, a straight chain or branched C₁-C₄ alkyl, a straight chain or branched C₁-C₄ alkyl substituted with one or more halogen atoms and/or hydroxy groups, halogen, straight chain or branched C₁-C₄ alkoxy,
R₇ is hydrogen, hydroxy, straight chain or branched C₁-C₄ alkyl, straight chain or branched C₁-C₄ alkoxy and
R₈ and R₉ are independently hydrogen, C₁-C₄ alkyl or form together a vinylene (-CH=CH-) or an ethylene (-CH₂CH₂-) radical, and
enantiomers, salts and solvates thereof.

Particularly preferred are the compounds wherein:
R₁ is methyl, R₄ is methoxy, R₂, R₃, R₅, R₆, R₈, R₉ are hydrogen, R₇ is hydroxy and n=1 (formoterol), and
R₁ is methyl, R₄ is methoxy, R₂, R₃, R₅, R₆ are hydrogen, R₇ is hydroxy, R₈ and R₉ together form a vinylene (-CH=CH-) radical and n=1.

The most preferred β₂ agonist of formula (I) is 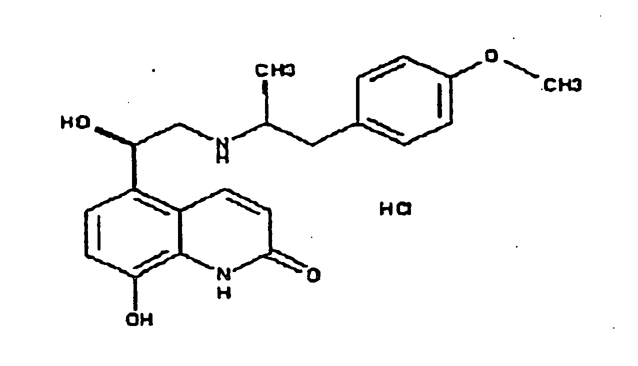 8-hydroxy-5-[(1R)-1-hydroxy-2-[[(1R)-2-(4-methoxyphenyl)-1-methylethyl]
amino]ethyl]-2(1H)-quinolinone hydrochloride also known with the experimental codes of TA 2005 and CHF 4226.

TA 2005 is highly potent and its dosage is considerably less than many other drugs which can be administered by MDls. Thus, its concentration in the aerosol formulation is very low and this factor, together with its chemico-physical properties, lead to problems in manufacturing and formulating a formulation which is stable and provides good dosage reproducibility when administered by MDls.

Furthermore, as mentioned above, the formulation of the invention contains a liquefied propellant.

Since the halogenated propellants have been banned as known to deplete the ozone layer, suitable propellant systems are considered hydrofluorocarbons (HFC or HFA) which are alkyl molecules with fluoro and hydrogen moieties on the carbon backbone. The formulation of the invention contains a liquefied propellant, namely a HFA propellant, selected from HFA 134a (1,1,1,2-tetrafluoroethane) and HFA 227 (1,1,1,2,3,3,3-heptafluoropropane) and mixtures thereof.

The formulation is preferably a solution in which the active ingredient is completely dissolved.

To this end it will include an adjuvant having a higher polarity than the propellant as a co-solvent to solubilize the active ingredient in the propellant. The co-solvent is preferably an alcohol; the most preferred is ethanol. It will be present in an amount suitable to solubilize the active ingredient in the propellant in a concentration comprised between 6% and 30%, preferably between 8% and 25%, more preferably between 10% and 20% by weight, based on the weight of the formulation.

Compositions of this kind have been described in the previous patent applications of the applicant, EP 1 157 689 ('689) filed on 18/05/2001, WO 03/074024 (024) filed on 26102/2003 and WO 03/074025 ('025) filed on 27/02/2003.

As disclosed in these previous applications, the active substances of the invention in solution in the HFA propellant/cosolvent system meet problems of chemical stability and can be stabilized by addition of strong mineral acids, preferably selected from hydrochloric, phosphoric, nitric and sulfuric acid. Phosphoric acid is preferred for the stabilization of TA 2005, in particular concentrated phosphoric acid such as 15 M phosphoric acid. Preferably phosphoric acid is contained in the medicinal aerosol formulation in an amount equivalent to 0.001 to 0.040 % w/w, more preferably 0.004 to 0.027 % w/w of 15 M phosphoric acid, based on the total weight of the formulation.

In the '689 application stability data of a HFA 134a solution formulation containing 8-hydroxy-5-[(1R)-1-hydroxy-2-[[(1R)-2-(4-methoxy-phenyl)-1-methylethyl]amino]ethyl]-2(1H)-quinolinone hydrochloride (TA 2005) 3.5 µg/50 µl dose, 12% w/w ethanol, 1% w/w isopropyl myristate stabilised by different amounts of HCl 0.08M (1.0, 1.4 and 1.8 µl) were reported (Example 7).

The stability was determined on formulations stored upright at 50° C in aluminium canisters having the internal surface coated with teflon and fitted with commercial valves.

The formulations seemed to be provided with quite good stability. Nevertheless, when the present inventors repeated the test, they noticed a progressive degradation of the active ingredient in the formulation.

Moreover, the formulation exemplified in '689 contained isopropyl myristate as a low volatility compound in order to increase the MMAD (mass median aerodynamic diameter) of the delivered particles. It has been subsequently found that it would be highly advantageous to provide highly efficient TA 2005 formulations characterised by a deeper lung penetration by virtue of a significant fraction, of at least 30%, of fine particles, with a diameter equal or less than 1.1 µm. Therefore the low volatility compound should be avoided.

In the other previous application '025, stability data of a HFA solution formulation comprising 8-hydroxy-5-[(1R)-1-hydroxy-2-[[(1R)-2-(4-methoxy-phenyl)-1-methylethyl]amino]ethyl]-2(1H)-quinolinone hydrochloride (TA 2005) stabilized by HCl were reported.

The stability was determined on a formulation containing 4 µg/63 µl of the active ingredient, stored upright at 5° C in aluminium canisters having the internal surface coated with teflon and fitted with valves comprising EPDM (ethylene-propylene-diene) gaskets. In said refrigerated conditions, after nine months, the TA 2005 assay was higher than 95%.

However, it has then been found by the present inventors that in a lower concentration and in other storage conditions, the active ingredient in the formulation rapidly degraded. This happened, for example, when the cans of WO 03/074025 were stored at 40° C and 70% relative humidity.

689 also provided a HFA solution formulation comprising, as a β₂-agonist, formoterol and its derivatives, whose chemical stability was improved by addition of a small amount of 1.0 M hydrochloric acid.

In the second previous application '024 it has been further disclosed that formoterol and its derivatives, in highly efficient formulations comprising a particularly high fraction of particles equal to or less than about 1 µm, are extremely sensitive to the humidity and that an amount of water higher than 1500 ppm on the total weight of the formulation is detrimental for their chemical stability.

As said before, the formulations in form of a solution are preferred.

However, the formulation can also be in form of a suspension and then optionally will contain other accessory substances. Accessory substances include small quantities of adjuvant as valve lubricant or to reduce the deposition on the actuator orifice of the inhaler, so improving the reproducibility of the dose after repeated administrations by keeping "clean" the actuator orifice used for dispersing the formulation to a patient, and dispersing agents of common use in this kind of formulation chosen among surfactants, such as polyethoxylated surfactants, fluorinated surfactants, fatty acids, their salts or esters of mono-, di- or triglycerides, sorbitan esters, phospholipids, alkylsaccharides, quaternary ammonium salts, oils or micronised bulking agents such as lactose, alanine, ascorbic acid and others.

According to the first aspect of the present invention it has been found that the stability of formulations of compounds of formula (I) in a solution of a HFA propellant is enhanced when stored in MDI containers fitted with valves provided with sealing rings and/or gaskets comprising an elastomeric material including particular kinds of butyl rubbers.

In EP 1 157 689 of the present applicant it is generically stated that metering valves fitted with gaskets made of chloroprene-based rubbers can preferably be used to reduce the ingress of moisture which can adversely affect the stability of the drug (page 5 lines 13-14). Furthermore, in WO 03/074 025 of the present applicant butyl rubbers are listed among many other suitable elastomeric materials for gaskets. EPDM (ethylene-propylene-diene monomer) rubbers and TPE (thermoplastic elastomer) are preferred. EPDM are particularly preferred (page 16, lines 8-12).

However, it has now been found by the present inventors that the active substances of formula (I) of the invention dissolved in a solution of a HFA propellant and a co-solvent further comprising a mineral acid, when stored in cans filled with valves having a gasket and/or sealing rings made of an elastomeric material including particular kinds of butyl rubbers of the type described in WO 03/078538, have a good chemical stability and meet the requirements of the ICH Guideline Q1A referring to "Stability Testing of new Active Substances (and Medicinal Products)", wherein a significant change for a drug product is defined as a 5 % change in assay from its initial value.

The sealing rings and/or gaskets for the metering valve for use in the pressurized metered dose inhaler of the medicinal aerosol formulation product according to the present invention are made of a vulcanisate of an elastomeric composition of a butyl rubber, a cross-linking agent for the butyl rubber, and an accelerator for the cross-linking agent, wherein the accelerator includes a polysulphide compound derived from a substituted dithiocarbonic acid or derivative thereof.

Butyl rubber is a copolymer made from isobutylene and a small amount of a diolefin, such as isoprene (2-methylbuta-1,3-diene). Typically, according to the present invention, butyl rubber comprises approximately 97% isobutylene and approximately 3% isoprene, and it may be polymerised using an aluminium chloride catalyst.

For the purposes of the present invention, particularly preferred are the halogenated butyl rubbers of the above-referenced composition (approx. 97 % isobutylene and approx. 3 % isoprene) among which bromobutyl rubbers are the most preferred.

The cross-linking agent (also known as the curing agent) provides or facilitates network formation to result in a three-dimensional polymer network structure. The cross-linking agent may act by reacting with the functional groups of the polymer chain. The cross-linking agent will typically comprise sulphur or a sulphur-containing compound. The cross-linking agent is preferably substantially free of any peroxide curing agents such as dicumyl peroxide.

The polysulphide compound used as the accelerator is preferably derived from a substituted xanthic acid or a derivative thereof, preferably of the type ROC(S)SH, in which R is typically an C₁-C₆ alkyl radical. The substituted group in the polysulphide compound typically comprises an isopropyl group.

The polysulphide compound preferably comprises three or more bridging sulphur atoms, more preferably 3, 4 or 5 bridging sulphur atoms.

The polysulphide compound is preferably substantially free of nitrogen, phosphorous and metallic elements.

Advantageously, the polysulphide compound comprises or consists of diisopropyl xanthogen polysulphide.

The elastomeric composition for preparing the vulcanisate typically comprises up to 3 % by weight of the accelerator based on the total weight of the accelerator and butyl rubber in the composition, more typically up to 1.5 % by weight of the accelerator based on the total weight of the accelerator and butyl rubber in the composition, still more typically up to 1- % by weight of the accelerator based on a total weight of the accelerator and butyl rubber.

The weight ratio of the accelerator to the cross-linking agent in the elastomeric composition is preferably in the range of from 1:1 to 3:1, more preferably from 1:1 to 2:1.

The sealing rings and/or gaskets may further include a filler, preferably a mineral filler, a process aid, preferably a low molecular weight polyethylene and further auxiliary ingredients as defined on page 9, line 28 to page 10, line 26 of WO 03/078538.

The sealing rings and/or gaskets of the metering valve may be provided as a separate component or may be formed integrally with the valve.

Preferably, the rubbers are extracted with a suitable pharmaceutically acceptable solvent, preferably warm ethanol, before their assembling in the metered dose inhaler. In general, solvents which are pharmaceutically acceptable and endowed with adequate capacity of extraction of oxides and peroxides can be utilised.

The demonstration of TA 2005 stability is offered in the Example 1.

### Example 1

A formulation for delivering a nominal dose of 1 µg per actuation of TA 2005 was prepared with the composition as follows:

| *Components* | *Amounts per unit* | |
|---|---|---|
| | mg | % |
| TA 2005 (1µg/63µl) | 0.154 | 0.0016 w/v |
| Ethanol | 1650.0 | 15.00 w/w |
| Phosphoric.acid 15M | 1.00 | 0.009 w/w |
| HFA 134a q.s. to 9.72 ml | 9348.8 | - |

Analogously, formulations able of delivering a nominal dose of 0.5, 1.5, 2, 2.5, 3, 3.5 or 4 µg of active ingredient per actuation can be prepared.

The formulation (120 actuations/canister, overage of 30 actuations) was filled in aluminium canisters having the internal surface coated with Teflon (two stage pressure filling) and fitted with a metering valve having a 63 µl metering chamber provided with a butyl rubber gasket as described in WO 03/078538 (Bespak) cited above. In particular, the gaskets were made of a bromobutyl rubber made of approximately 97% isobutylene and approximately 3% isoprene and having been polymerised by using an aluminium chloride catalyst and by treating the thus obtained isoprene-isobutylene rubber with bromine.

The same formulation was filled in the same kind of cans fitted with valves provided with EPDM rubber gaskets.

A stability study was carried out storing the formulation in upright and inverted cans at 40° C and 75% relative humidity.

After three months the percent amount of TA 2005 both in upright and inverted cans fitted with the valves provided with bromobutyl rubber gaskets was 98 and 97%, respectively.

On the contrary the percent amount of TA 2005 in the formulations stored in upright and inverted cans fitted with valves provided with EPDM rubber gaskets was 98 and 77%, respectively. The results were obtained as a mean of two cans.

The demonstration of formoterol fumarate stability is offered in the Example 2.

### Example 2

A formulation for delivering a nominal dose of 12 µg per actuation of formoterol fumarate was prepared with the composition as follows:

| Components | Amounts per unit | |
|---|---|---|
| | mg | % |
| Formoterol fumarate | 1.92 | |
| (12µg/63µl) | | 0.019 w/v |
| Ethanol | 1387.2 | 12.00 w/w |
| Hydrochloric acid 1 M | 4.3 | 0.037 w/w |
| HFA 134a | 10166.58 | |

The formulation (120 actuations/canister, overage of 40 actuations) was filled in standard aluminium canisters and fitted with a metering valve having a 63 µl metering chamber provided with a butyl rubber gasket as described in WO 03/078538 (Bespak) cited above. In particular, the gaskets were made of a bromobutyl rubber made of approximately 97% isobutylene and approximately 3% isoprene and having been polymerised by using an aluminium chloride catalyst and by treating the thus obtained isoprene-isobutylene rubber with bromine.

The same formulation was filled in the same kind of cans fitted with valves provided with EPDM rubber gaskets.

A stability study was carried out storing the formulation in upright and inverted cans at 25° C.

After three months the percent amount of formoterol fumarate both in upright and inverted cans fitted with the valves provided with bromobutyl rubber gaskets was 98 and 97%, respectively.

On the contrary the percent amount of formoterol fumarate in the formulations stored in upright and inverted cans fitted with valves provided with EPDM rubber gaskets was 94 and 93%, respectively, already after 31 days storage at 40° C. The results were obtained as a mean of two cans.

The results show that the valve material affects the chemical stability of compounds of formula (I) and valves provided with specific bromobutyl rubber gaskets improve the stability of said compounds in HFA solution formulations.

The stability test in Examples 1 and 2 was carried out both in upright and inverted cans. In inverted position, the formulation is in direct contact with the valve materials for all the duration of the test in order to detect possible chemical interactions with the valve materials that may negatively affect the stability of the active ingredient in the formulation. In upright cans the interactions between the valve materials and the formulation are very limited and so also the possible negative effects on the stability of the active ingredient in the formulation may be unnoticed.

As shown in Example 2, the metering valve of the invention provided with a sealing ring and/or gasket comprising an elastomeric material including particular kinds of bromobutyl rubbers can be advantageously utilized also for metered dose inhalers filled with a medicinal formulation comprising formoterol or its derivatives in a solution consisting of a hydrofluorocarbon propellant, a co-solvent and a mineral acid. For formoterol derivatives the preferred mineral acid is hydrochloric acid, in particular 1.0 M hydrochloric acid and for a formulation delivering a dose of 12 µg per actuation the amount of 1.0 M hydrochloric acid is of 0.020 to 0.050 %, preferably of 0.025 to 0.045 % by volume on the total volume of the formulation, corresponding to an amount of 0.030 to 0.045 %, preferably of 0.035 to 0.040 % by weight on the total weight of the formulation.

In fact, it has been found that the sealing rings and/or gaskets for the metering valve made of an elastomeric material including particular kinds of butyl rubbers of the type described in WO 03/078538 may also improve the chemical stability of formoterol fumarate, probably due to the hermetic sealing-off of the pressurized canister, providing a better protection of the formulation from the environmental moisture ingress which is detrimental to the chemical stability of the compound.

The stability of the compounds of formula (I) may be affected also by the presence of metal iones released from the metal parts of the valve, constituted in particular by the spring which can come into contact with the formulation.
For this reason springs made of a stainless steel alloy containing titanium are particularly preferred.

In summary, the present invention provides a medicinal aerosol formulation product with improved chemical stability, comprising a pressurized metered dose inhaler (MDI) by using a specific butyl rubber as a material for the sealing rings and/or gaskets in the metering valve and by using an aerosol formulation specifically stabilized with a suitable mineral acid.

## Claims

1. A medicinal aerosol formulation product with improved chemical stability, comprising
a pressurised metered dose inhaler, comprising an aerosol canister equipped with a metering valve provided with sealing rings and/or gaskets made of a vulcanisate of an elastomeric composition of a butyl rubber, a cross-linking agent for the butyl rubber, and an accelerator for the cross-linking agent, wherein the accelerator includes a polysulphide compound derived from a substituted dithiocarbonic acid or derivative thereof,
wherein the aerosol canister contains a medicinal aerosol formulation containing a long acting β₂-agonist of formula (I) 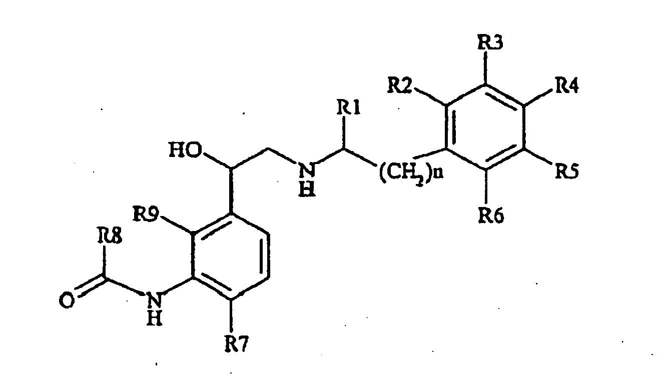 wherein R1 is methyl and R2 is hydrogen or R1 and R2 form a methylenic bridge -(CH₂)n- with n is 1 or 2,
R3, R4, R5 and R6 are each independently hydrogen, hydroxy, a straight chain or branched C1-C4 alkyl, a straight chain or branched C1-C4 alkyl substituted with one or more halogen atoms and/or hydroxy groups, halogen, straight chain or branched C1-C4 alkoxy,
R7 is hydrogen, hydroxy, straight chain or branched C1-C4 alkyl, straight chain or branched C1-C4 alkoxy, and
R8 and R9 are independently hydrogen, C1-C4 alkyl or form together a vinylene (-CH=CH-) or an ethylene (-CH2-CH2-) radical, and
enantiomers, salts and solvates thereof,
a hydrofluorocarbon propellant,
a co-solvent, and
a mineral acid as a stabilizer for the active ingredient.

2. Medicinal aerosol formulation product according to claim 1, wherein in formula (I) R1 is methyl, R4 is methoxy and R2, R3, R5, R6, R8 and R9 are hydrogen, R7 is hydroxy and n = 1.

3. Medicinal aerosol formulation product according to claim 1, wherein in the formula (I) R1 is methyl, R4 is methoxy, R2, R3, R5 and R6 are hydrogen, R7 is hydroxy, R8 and R9 together form a vinylene (-CH=CH-) radical and n =1.

4. Medicinal aerosol formulation product according to any one of claims 1 to 3,
wherein the medicinal aerosol formulation is a medicinal aerosol solution formulation.

5. Medicinal aerosol formulation product according to any one of claims 1 to 3,
wherein the medicinal aerosol formulation is a medicinal aerosol suspension formulation.

6. Medicinal aerosol formulation product according to claim 5, wherein the medicinal aerosol suspension formulation contains valve lubricants and/or dispersing agents.

7. Medicinal aerosol formulation product according to any one of claims 1 to 6,
wherein the hydrofluorocarbon propellant is selected from the group consisting of HFA 134a, HFA 227 and mixtures thereof.

8. Medicinal aerosol formulation product according to any one of claims 1 to 4,
wherein the co-solvent is ethanol.

9. Medicinal aerosol formulation product according to any one of claims 1 to 8,
wherein part or all of the internal surfaces of said pressurised metered dose inhaler consists of stainless steel, anodised aluminium or is lined with an inert organic coating.

10. Medicinal aerosol formulation product according to claim 9, wherein the inert organic coating is a perfluoroalkoxyalkane, a perfluoroalkoxyalkylene, a perfluoroalkylene, such as polytetrafluoroethylene, epoxy-phenol resin or fluorinated-ethylene-propylene, polyether sulfone or a combination thereof.

11. Medicinal aerosol formulation product according to any one of claims 1 to 10, wherein the metering valve comprises a valve spring made of a stainless steel alloy containing titanium.

12. Medicinal aerosol formulation product according to any one of claims 1 to 11, wherein the sealing rings and/or gaskets of the valve are extracted with ethanol, preferably warm ethanol before use.

13. Medicinal aerosol formulation product according to claim 2, wherein the mineral acid is hydrochloric acid, in particular 1 M hydrochloric acid, and is contained in the medicinal aerosol formulation in an amount equivalent to 0.030 to 0.045 % w/w of 1 M hydrochloric acid, based on the total weight of the formulation.

14. Medicinal aerosol formulation product according to claim 3, wherein the mineral acid is phosphoric acid, in particular 15 M phosphoric acid, and is contained in the medicinal aerosol formulation in an amount equivalent to 0.001 to 0.040% w/w of 15 M phosphoric acid, based on the total weight of the formulation.

15. Medicinal aerosol formulation product according to any one of claims 1 to 13, wherein the butyl rubber is made of 97% isobutylene and 3% isoprene and being polymerised by using an aluminium chloride catalyst.

16. Medicinal aerosol formulation product according to claim 15 wherein the butyl rubber is a bromobutyl rubber.

17. Medicinal aerosol formulation product according to any one of claims 1 to 16, wherein the cross-linking agent comprises sulphur or a sulphur-donating compound.

18. Medicinal aerosol formulation product according to any one of claims 1 to 17, wherein said polysulphide compound is derived from a substituted xanthic acid or a derivative thereof.

19. Medicinal aerosol formulation product according to any one of claims 1 to 18, wherein the substituted group in said polysulphide compound comprises or consists of an isopropyl group.

20. Medicinal aerosol formulation product according to any one of claims 1 to 19, wherein said polysulphide compound comprises or consists of diisopropyl xanthogen polysulphide.

21. Medicinal aerosol formulation product according to any one of claims 1 to 20, wherein said polysulphide compound comprises three or more bridging sulphur atoms.

22. Medicinal aerosol formulation product according to any one of claims 1 to 21; wherein said polysulphide compound is free of nitrogen, phosphorous and metallic elements.

23. Medicinal aerosol formulation product according to any one of claims 1 to 22, wherein the elastomeric composition comprises up to 3 % by weight of the accelerator based on the total weight of the accelerator and the butyl rubber in the composition.

24. Medicinal aerosol formulation product according to any one of claims 1 to 22, wherein the elastomeric composition comprises up to 1.5 % by weight of the accelerator based on the total weight of the accelerator and the butyl rubber in the composition.

25. Medicinal aerosol formulation product according to any one of claims 1 to 24, wherein the weight ratio of the accelerator to the cross-linking agent in the elastomeric composition is in the range of from 1:1 to 3:1.

## Patentansprüche

1. Medizinisches Aerosolformulierungsprodukt mit verbesserter chemischer Stabilität, umfassend
ein Treibgasdosieraerosol-Inhalator, umfassend eine Aerosoldose, die mit einem Dosierventil ausgestattet ist, welches mit Dichtungsringen und/oder Dichtungsmanschetten versehen ist, welche aus einem Vulkanisat einer Elastomerzusammensetzung aus einem Butylkautschuk, einem Vernetzungsmittel für den Butylkautschuk und einem Beschleuniger für das Vernetzungsmittel hergestellt sind, wobei der Beschleuniger eine Polysulfidverbindung, die von einer substituierten Dithiocarbonsäure oder einem Derivat davon abgeleitet ist, umfasst, wobei die Aerosoldose eine medizinische Aerosolformulierung enthält, enthaltend einen langwirkenden β₂-Agonisten der Formel (I) 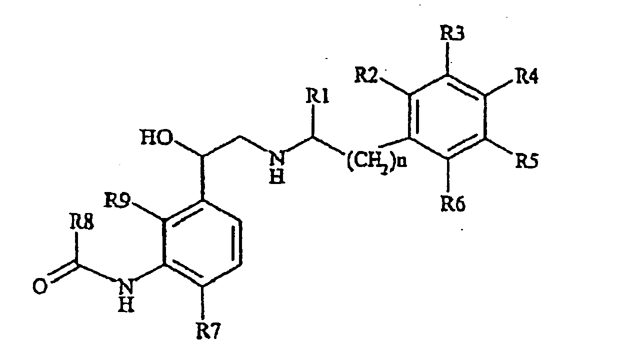 worin R1 Methyl ist und R2 Wasserstoff ist oder R1 und R2 eine Methylenbrücke -(CH₂)ₙ-, worin n 1 oder 2 ist, bilden; R3, R4, R5 und R6 jeweils unabhängig Wasserstoff, Hydroxy, ein geradkettiges oder verzweigtes C1-C4-Alkyl, ein geradkettiges oder verzweigtes C1-C4-Alkyl, substituiert mit einem oder mehreren Halogenatom(en) und/oder einer oder mehreren Hydroxygruppe(n), Halogen, geradkettiges oder verzweigtes C1-C4-Alkoxy sind;
R7 Wasserstoff, Hydroxy, geradkettiges oder verzweigtes C1-C4-Alkyl, geradkettiges oder verzweigtes C1-C4-Alkoxy ist und R8 und R9 jeweils unabhängig Wasserstoff, C1-C4-Alkyl sind oder zusammen einen Vinylen (-CH=CH-) oder einen Ethylen (-CH2-CH2-)-Rest bilden, und
Enantiomere, Salze und Solvate davon,
ein Fluorkohlenwasserstofftreibmittel,
ein Colösungsmittel und
eine Mineralsäure als Stabilisator für das aktive Ingredienz.

2. Medizinisches Aerosolformulierungsprodukt nach Anspruch 1, wobei in Formel (I) R1 Methyl ist, R4 Methoxy ist und R2, R3, R5, R6, R8 und R9 Wasserstoff sind, R7 Hydroxy ist und n gleich 1 ist.

3. Medizinisches Aerosolformulierungsprodukt nach Anspruch 1, wobei in der Formel (I) R1 Methyl ist, R4 Methoxy ist, R2, R3, R5 und R6 Wasserstoff sind, R7 Hydroxy ist, R8 und R9 zusammen einen Vinylen (-CH=CH-)-Rest bilden und n gleich 1 ist.

4. Medizinisches Aerosolformulierungsprodukt nach einem der Ansprüche 1 bis 3, wobei die medizinische Aerosolformulierung eine medizinische Aerosollösungsformulierung ist.

5. Medizinisches Aerosolformulierungsprodukt nach einem der Ansprüche 1 bis 3, wobei die medizinische Aerosolformulierung eine medizinische Aerosolsuspensionsformulierung ist.

6. Medizinisches Aerosolformulierungsprodukt nach Anspruch 5, wobei die medizinische Aerosolsuspensionsformulierung Ventil-Schmiermittel und/oder Dispergiermittel enthält.

7. Medizinisches Aerosolformulierungsprodukt nach einem der Ansprüche 1 bis 6, wobei das Fluorkohlenwasserstofftreibmittel aus der Gruppe, bestehend aus HFA 134a, HFA 227 und Gemischen davon, ausgewählt ist.

8. Medizinisches Aerosolformulierungsprodukt nach einem der Ansprüche 1 bis 4, wobei das Colösungsmittel Ethanol ist.

9. Medizinisches Aerosolformulierungsprodukt nach einem der Ansprüche 1 bis 8, wobei die inneren Oberflächen des Treibgasdosieraerosol-Inhalators ganz oder teilweise aus Edelstahl, anodisiertem Aluminium bestehen oder mit einer inerten organischen Beschichtung versehen sind.

10. Medizinisches Aerosolformulierungsprodukt nach Anspruch 9, wobei die inerte organische Beschichtung ein Perfluoralkoxyalkan, ein Perfluoralkoxyalkylen, ein Perfluoralkylen, zum Beispiel Polytetrafluorethylen, Epoxyphenolharz oder fluoriertes Ethylen-Propylen, Polyethersulfon oder eine Kombination davon ist.

11. Medizinisches Aerosolformulierungsprodukt nach einem der Ansprüche 1 bis 10, wobei das Dosierventil eine Ventilfeder umfasst, die aus einer titanenthaltenden Edelstahllegierung hergestellt ist.

12. Medizinisches Aerosolformulierungsprodukt nach einem der Ansprüche 1 bis 11, wobei die Dichtungsringe und/oder Dichtungsmanschetten des Ventils vor Verwendung mit Ethanol, vorzugsweise warmem Ethanol, extrahiert wurden.

13. Medizinisches Aerosolformulierungsprodukt nach Anspruch 2, wobei die Mineralsäure Salzsäure, insbesondere 1 M Salzsäure, ist und in der medizinischen Aerosolformulierung in einer Menge, die 0,03 bis 0,045% G/G 1 M Salzsäure äquivalent ist, bezogen auf das Gesamtgewicht der Formulierung, enthalten ist.

14. Medizinisches Aerosolformulierungsprodukt nach Anspruch 3, wobei die Mineralsäure Phosphorsäure, insbesondere 15 M Phosphorsäure, ist und in der medizinischen Aerosolformulierung in einer Menge enthalten ist, die 0,001 bis 0,040% G/G 15 M Phosphorsäure äquivalent ist, bezogen auf das Gesamtgewicht der Formulierung.

15. Medizinisches Aerosolformulierungsprodukt nach einem der Ansprüche 1 bis 13, wobei der Butylkautschuk aus 97% Isobutylen und 3% Isopren besteht und unter Verwendung eines Aluminiumchloridkatalysators polymerisiert wird.

16. Medizinisches Aerosolformulierungsprodukt nach Anspruch 15, wobei der Butylkautschuk ein Brombutylkautschuk ist.

17. Medizinisches Aerosolformulierungsprodukt nach einem der Ansprüche 1 bis 16, wobei das Vernetzungsmittel Schwefel oder eine schwefelliefernde Verbindung umfasst.

18. Medizinisches Aerosolformulierungsprodukt nach einem der Ansprüche 1 bis 17, wobei die Polysulfidverbindung von einer substituierten Xanthosäure oder einem Derivat davon abgeleitet ist.

19. Medizinisches Aerosolformulierungsprodukt nach einem der Ansprüche 1 bis 18, wobei die substituierte Gruppe in der genannten Polysulfidverbindung eine Isopropylgruppe umfasst oder aus einer Isopropylgruppe besteht.

20. Medizinisches Aerosolformulierungsprodukt nach einem der Ansprüche 1 bis 19, wobei die Polysulfidverbindung Diisopropylxanthogenpolysulfid umfasst oder aus Diisopropylxanthogenpolysulfid besteht.

21. Medizinisches Aerosolformulierungsprodukt nach einem der Ansprüche 1 bis 20, wobei die Polysulfidverbindung drei oder mehr brückenbildende Schwefelatome umfasst.

22. Medizinisches Aerosolformulierungsprodukt nach einem der Ansprüche 1 bis 21, wobei die Polysulfidverbindung frei von Stickstoff, Phosphor und Metallelementen ist.

23. Medizinisches Aerosolformulierungsprodukt nach einem der Ansprüche 1 bis 22, wobei die Elastomerzusammensetzung bis zu 3 Gew.-% des Beschleunigers, bezogen auf das Gesamtgewicht des Beschleunigers und des Butylkautschuks in der Zusammensetzung, umfasst.

24. Medizinisches Aerosolformulierungsprodukt nach einem der Ansprüche 1 bis 22, wobei die Elastomerzusammensetzung bis zu 1,5 Gew.-% des Beschleunigers, bezogen auf das Gesamtgewicht des Beschleunigers und des Butylkautschuks in der Zusammensetzung, umfasst.

25. Medizinisches Aerosolformulierungsprodukt nach einem der Ansprüche 1 bis 24, wobei das Gewichtsverhältnis des Beschleunigers zu dem Vernetzungsmittel in der Elastomerzusammensetzung im Bereich von 1:1 bis 3:1 ist.

## Revendications

1. Un produit de formulation d'aérosol médicinal avec une stabilité chimique améliorée, comportant
un aérosol-doseur pressurisé, comportant une boîte d'aérosol équipée d'une valve doseuse équipée par des joints d'étanchéité et/ou des garnitures fabriquées d'un vulcanisate de composition d'élastomère d'un caoutchouc en butyle, d'un agent de réticulation pour le caoutchouc en butyle, et d'un accélérateur pour l'agent de réticulation, où l'accélérateur inclut un composé de polysulfure dérivé d'un acide dithiocarbonique substitué ou son dérivé, où la boîte d'aérosol contient une formulation d'aérosol médicinale un agonistes β₂ agissant à long terme de la formule (I) 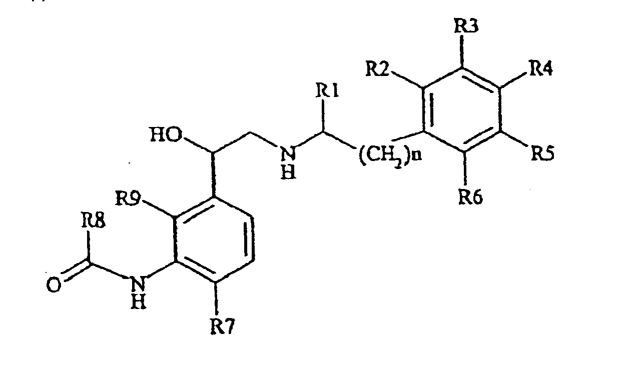 où R1 est un méthyle et R2 est hydrogène ou R1 et R2 forment un pont methylénique - (CH2)n- avec n est 1 ou 2,
R3, R4, R5 et R6 sont chacun indépendamment de l'hydrogène, hydroxy, une chaîne droite ou un alkyle C1-C4 ramifié, une chaîne droite ou un alkyle C1-C4 ramifié substitué avec un ou plusieurs atomes d'halogène et/ou groupes hydroxy, l'halogène, la chaîne droite ou l'acloxy C1-C4 ramifié,
R7 est hydrogène, hydroxy, chaîne droite ou ramifiée d'alkyle C1-C4 ou chaîne droite ou ramifiée d'alkoxy C1-C4, et
R8 et R9 sont indépendamment hydrogène, l'alkyle C1-C4 ou forment à la fois un radical vinylène (- CH=CH -) ou éthylène (- CH2-CH2 -), et
énantiomères, sels et ses solvates,
un propulseur d'hydrofluorocarbon,
un co-dissolvant, et
un acide minéral comme stabilisateur pour l'ingrédient actif.

2. Un produit de formulation d'aérosol médicinal conformément à la revendication 1, où dans la formule (I) R1 est méthyle, R4 est méthoxy et R2, R3, R5, R6, R8 et R9 sont hydrogène, R7 est hydroxy et n = 1.

3. Un produit de formulation d'aérosol médicinal conformément à la revendication 1, où dans la formule (I) R1 est le méthyle, R4 est méthoxy, R2, R3, R5 et R6 sont hydrogène, R7 est hydroxy, R8 et R9 forment ensemble un radical vinylène (- CH=CH -) et n = 1.

4. Un produit de formulation d'aérosol médicinal conformément à quelconque des revendications 1 à 3, où la formulation médicinale d'aérosol est une formulation de solution d'aérosol médicinal.

5. Un produit de formulation d'aérosol médicinal conformément à quelconque des revendications 1 à 3, où la formulation médicinale d'aérosol est une formulation de suspension d'aérosol médicinal.

6. Un produit de formulation d'aérosol médicinal conformément à la revendication 5, où la formulation de suspension d'aérosol médicinal contient des lubrifiants de valve et/ou des agents de dispersion.

7. Un produit de formulation d'aérosol médicinal conformément à quelconque des revendications 1 à 6, où le propulseur d'hydro-fluoro-carbon est choisi dans le groupe HFA 134a, HFA 227 et de leurs mélanges.

8. Un produit de formulation d'aérosol médicinal conformément à quelconque des revendications 1 à 4, où le co-dissolvant est l'éthanol.

9. Un produit de formulation d'aérosol médicinal conformément à quelconque des revendications 1 à 8, où une partie ou toutes les surfaces internes de l'aérosol-doseur pressurisé précité se compose de l'acier inoxydable, de l'aluminium anodisé ou il est garni d'un enduit organique inerte.

10. Un produit de formulation d'aérosol médicinal conformément à la revendication 9, où l'enduit organique inerte est un perfluoroalkoxyalkane, un perfluoroalkoxyalkylene, un perfluoroalkylene, tel que le polytétrafluoroéthylène, la résine ou fluoré-éthylène-propylène d'époxyde-phénol, la sulfone de polyéther ou de sa combinaison.

11. Un produit de formulation d'aérosol médicinal conformément à quelconque des revendications 1 à 10, où la valve doseuse comporte un ressort de valve fait d'un alliage d'acier inoxydable contenant le titane.

12. Un produit de formulation d'aérosol médicinal conformément à quelconque des revendications 1 à 11. où les joints d'étanchéité et/ou les garnitures de la valve sont extraites au moyen de l'éthanol, de préférence l'éthanol chaud avant l'emploi.

13. Un produit de formulation d'aérosol médicinal conformément à la revendication 2, où l'acide minéral est l'acide chlorhydrique, en particulier l'acide chlorhydrique 1 M, et il est contenu dans la formulation d'aérosol médicinal dans une quantité équivalente à 0.030 à 0.045 % de w/w d'acide chlorhydrique 1 M, sur la base du poids total de la formulation.

14. Un produit de formulation d'aérosol médicinal conformément à la revendication 3, où l'acide minéral est l'acide phosphorique, en particulier l'acide phosphorique 15 M, et il est contenu dans la formulation d'aérosol médicinal dans une quantité équivalente de 0.001 à 0.040% w/w d'acide phosphorique 15 M, sur la base du poids total de la formulation.

15. Un produit de formulation d'aérosol médicinal conformément à quelconque de revendications 1 à 13, où le caoutchouc de butyle est fabriqué de 97% d'isobutylène et de 3% d'isoprène et en employant un catalyseur de chlorure d'aluminium.

16. Un produit de formulation d'aérosol médicinal conformément à la revendication 15 où le caoutchouc en butyle est un caoutchouc de bromobutyle.

17. Un produit de formulation d'aérosol médicinal conformément à quelconque des revendications de 1 à 16, où l'agent de réticulation comporte le soufre ou un composé donateur de soufre.

18. Un produit de formulation d'aérosol médicinal conformément à quelconque des revendications de 1 à 17, où le composé polysulfure précité est dérivé d'un acide xanthique substitué ou de son dérivé.

19. Un produit de formulation d'aérosol médicinal conformément à quelconque des revendications de 1 à 18, où le groupe substitué dans le composé polysulfure précité se compose ou consiste d'un groupe isopropyle.

20. Un produit de formulation d'aérosol médicinal conformément à quelconque de revendications de 1 à 19, où le composé polysulfure précité comporte ou se compose du polysulfure diisopropylique xanthogen.

21. Un produit de formulation d'aérosol médicinal conformément à quelconque de revendications de 1 à 20, où le composé polysulfure précité comporte trois atomes de pont ou plus de soufre.

22. Un produit de formulation d'aérosol médicinal conformément à quelconque des revendications de 1 à 21, où le composé de polysulfure précité est exempt d'azote, d'élément phosphoreux et métalliques.

23. Un produit de formulation d'aérosol médicinal conformément à quelconque des revendications de 1 à 22, où la composition élastomère comporte jusqu'à 3 % par poids de l'accélérateur sur la base du poids total de l'accélérateur et le caoutchouc en butyle dans la composition.

24. Un produit de formulation d'aérosol médicinal conformément à quelconque des revendications de 1 à 22, où la composition élastomère comporte jusqu'à 1.5 % par poids de l'accélérateur sur la base du poids total de l'accélérateur et le caoutchouc en butyle dans la composition.

25. Un produit de formulation d'aérosol médicinal conformément à quelconque des revendications de 1 à 24, où le rapport du poids de l'accélérateur à l'agent de réticulation dans la composition élastomère est dans la gamme de 1:1 à 3:1.
